# EUROPEAN PATENT SPECIFICATION

(11) **EP 2 030 075 B1**
(45) Date of publication and mention of the grant of the patent: **04.01.2017**
(21) Application number: 07784415.7
(22) Date of filing: 12.06.2007
(51) Int. Cl.: G02C 7/10

(54) **COLOR BALANCED OPHTHALMIC SYSTEM WITH SELECTIVE LIGHT INHIBITION**
FARBAUSGEGLICHENES AUGENSYSTEM MIT SELEKTIVER LICHTHEMMUNG
SYSTÈME OPHTALMIQUE ÉQUILIBRÉ EN COULEUR À INHIBITION DE LUMIÈRE SÉLECTIVE

(30) Priority: 12.06.2006 US 812628 P
(43) Date of publication of application: 04.03.2009
(73) Proprietor: High Performance Optics, Inc., Roanoke, Virginia 24018 (US)
(72) Inventor: ISHAK, Andrew, W., Waterford, Vermont 05819 (US); HADDOCK, Joshua, N., Roanoke, VA 24011 (US); KOKONASKI, William, Gig Harbor WA, WV 98335 (US); DUSTON, Dwight, Laguna Niguel, CA 92677 (US); IYER, Venkatramani, Roanoke, VA 24012 (US); BLUM, Ronald, D., Roanoke, VA 24014 (US)
(74) Representative: Vossius & Partner Patentanwälte Rechtsanwälte mbB
(86) International application number: PCT/US2007/070995
(87) International publication number: WO 2007/146933

(56) References cited:
- WO-A2-2007/109202
- US-A- 4 793 669
- US-A- 5 729 379
- US-A- 6 145 984
- US-A1- 2002 126 256
- US-A1- 2003 229 131
- US-A1- 2005 018 131
- US-B1- 6 373 615
- US-B2- 6 444 146

## Description

### BACKGROUND

Current research strongly supports the premise that short wavelength visible light (blue light) having a wavelength of approximately 400 nm - 500 nm (nanometers or 10⁻⁹ meters) could be a contributing cause of AMD (age related macular degeneration). It is believed that the highest level of blue light absorption occurs in a region around 430 nm, such as 400 nm - 460 nm. Research further suggests that blue light worsens other causative factors in AMD, such as heredity, tobacco smoke, and excessive alcohol consumption.

Light is made up of electromagnetic radiation that travels in waves. The electromagnetic spectrum includes radio waves, millimeter waves, microwaves, infrared, visible light, ultra-violet (UVA and UVB) and x-rays and gamma rays. The human retina responds only to the visible light portion of the electromagnetic spectrum. The visible light spectrum includes the longest visible light wavelength of approximately 700nm and the shortest of approximately 400nm. Blue light wavelengths fall in the approximate range of 400nm to 500nm. For the ultra-violet bands, UVB wavelengths are from 290nm to 320nm and UVA wavelengths are from 320nm to 400nm.

The human retina includes multiple layers. These layers listed in order from the first exposed to any light entering the eye to the deepest include:
1) Nerve Fiber Layer
2) Ganglion Cells
3) Inner Plexiform Layer
4) Bipolar and Horizontal Cells
5) Outer Plexiform Layer
6) Photoreceptors (Rods and Cones)
7) Retinal Pigment Epithelium (RPE)
8) Bruch's Membrane
9) Choroid

When light is absorbed by the eye's photoreceptor cells, (rods and cones) the cells bleach and become unreceptive until they recover. This recovery process is a metabolic process and is called the "visual cycle." Absorption of blue light has been shown to reverse this process prematurely. This premature reversal increases the risk of oxidative damage and is believed to lead to the buildup of the pigment lipofuscin in the retina. This build up occurs in the retinal pigment epithelium (RPE) layer. It is believed that aggregates of extra-cellular materials called drusen are formed in the RPE layer due to the excessive amounts of lipofuscin. Drusen hinder or block the RPE layer from providing the proper nutrients to the photoreceptors, which leads to damage or even death of these cells. To further complicate this process it appears that when lipofuscin absorbs blue light in high quantities it becomes toxic, causing further damage and/or death of the RPE cells. It is believed that the lipofuscin constituent A2E is at least partly responsible for the short-wavelength sensitivity of RPE cells. A2E has been shown to be maximally excited by blue light; the photochemical events resulting from such excitation can lead to cell death. See, for example, Janet R. Sparrow et al., "Blue light-absorbing intraocular lens and retinal pigment epithelium protection in vitro," J. Cataract Refract. Surg. 2004, vol. 30, pp. 873-78.

The lighting and vision care industries have standards as to human vision exposure to UVA and UVB radiation Surprisingly, no such standard is in place with regard to blue light. For example, in the common fluorescent tubes available today, the glass envelope mostly blocks ultra-violet light but blue light is transmitted with little attenuation. In some cases, the envelope is designed to have enhanced transmission in the blue region of the spectrum.

Ophthalmic systems that provide blue blocking to some degree are known.
However, there are disadvantages associated with such systems. For example, they tend to be cosmetically unappealing because of a yellow or amber tint that is produced in lenses by blue blocking. More specifically, one common technique for blue blocking involves tinting or dyeing lenses with a blue blocking tint, such as BPI Filter Vision 450 or BPI Diamond Dye 500. The tinting may be accomplished, for example, by immersing the lens in a heated tint pot containing a blue blocking dye solution for some predetermined period of time. Typically, the dye solution has a yellow or amber color and thus imparts a yellow or amber tint to the lens. To many people, the appearance of this yellow or amber tint may be undesirable cosmetically. Moreover, the tint may interfere with the normal color perception of a lens user, making it difficult, for example, to correctly perceive the color of a traffic light or sign.

Efforts have been made to compensate for the yellowing effect of conventional blue blocking filters. For example, blue blocking lenses have been treated with additional dyes, such as blue, red or green dyes, to offset the yellowing effect. The treatment causes the additional dyes to become intermixed with the original blue blocking dyes. However, while this technique may reduce yellow in a blue blocked lens, intermixing of the dyes may reduce the effectiveness of the blue blocking by allowing more of the blue light spectrum through. Moreover, these conventional techniques undesirably reduce the overall transmission of light wavelengths other than blue light wavelengths. This unwanted reduction may in turn result in reduced visual acuity for a lens user.

In view of the foregoing, there is a need for an ophthalmic system that allows for selective blockage of wavelengths of blue light while at the same time transmitting in excess of 80% of visible light and being perceived as mostly color neutral by someone observing the ophthalmic system when worn by a wearer. In addition, it is further important that such a system not impair the wearer's color vision and further that reflections from the back surface of the system into the eye of the wearer be at a level of not being objectionable to the wearer. This need exists as more and more data is pointing to blue light as one of the possible contributory factors in macula degeneration (the leading cause of blindness in the industrialized world) and also other retinal diseases.

WO 2007/109202 A2 describes an ophthalmic system combining ophthalmic components with blue light wavelength blocking and color-balancing functionalities. US 6,373,615 describes a neutral-color gray photochromic plastic. article which is designed to remain almost completely color-neutral or gray both when darkening and when fading. US 5,729,379. describes an electrochromic device having a conducting electrode opposing a counter conducting electrode with an electrochemically active polymer layer disposed on an opposing surface of one of the electrodes and an electrolyte containing at least one redox active material contactingly disposed between the electrochemically active layer and an other opposing surface of one of said electrodes.

### SUMMARY OF THE INVENTION

The present invention relates to an ophthalmic system. More particularly, the invention relates to an ophthalmic system that performs blocking of blue light wavelengths, while presenting a cosmetically attractive product.

An ophthalmic system is provided that can provide 80% or better transmission of visible light, inhibit selective wavelengths of blue light, allow for the wearer's proper color vision performance, and provide a mostly color neutral appearance to an observer looking at the wearer wearing such a lens or lens system. The system may use various optical coatings, films, materials, and absorbing dyes to produce the desired effect.

### BRIEF DESCRIPTION OF THE DRAWINGS

FIGS. 1A and 1B show examples of an ophthalmic system including a posterior blue blocking component and an anterior color balancing component.
FIG. 2 shows an example of using a dye resist to form an ophthalmic system.
FIG. 3 illustrates an exemplary system with a blue blocking component and a color balancing component integrated into a clear or mostly clear ophthalmic lens.
FIG. 4 illustrates an exemplary ophthalmic system formed using an in-mold coating.
FIG. 5 illustrates the bonding of two ophthalmic components.
FIG. 6 illustrates exemplary ophthalmic systems using anti-reflective coatings.
FIGS. 7A-7C illustrate various exemplary combinations of a blue blocking component, a color balancing component, and an ophthalmic component.
FIGS. 8A and 8B show examples of an ophthalmic system including a multifunctional blue blocking and color-balancing component.
FIG. 9 shows a reference of observed colors that correspond to various CIE coordinates.
FIG. 10 shows the transmission of the Gentext E46S absorbing dye.
FIG. 11 shows the absorbance of the Gentex E465 absorbing dye.
FIG. 12 shows the transmittance of a polycarbonate substrate with a dye concentration suitable for absorbing in the 430nm range.
FIG. 13 shows the transmittance as a function of wavelength of a polycarbonate substrate with an antireflective coating.
FIG. 14 shows the color plot of a polycarbonate substrate with an antireflective coating.
FIG. 15 shows the transmittance as a function of wavelength of an uncoated polycarbonate substrate and a polycarbonate substrate with an antireflective coating on both surfaces.
FIG. 16 shows the spectral transmittance of a 106 nm layer of TiO₂ on a polycarbonate substrate.
FIG. 17 shows the color plot of a 106 nm layer of TiO₂ on a polycarbonate substrate.
FIG. 18 shows the spectral transmittance of a 134 nm layer of TiO₂ on a polycarbonate substrate.
FIG. 19 shows the color plot of a 134 nm layer of TiO₂ on a polycarbonate substrate.
FIG. 20 shows the spectral transmittance of a modified AR coating suitable for color balancing a substrate having a blue absorbing dye.
FIG. 21 shows the color plot of a modified AR coating suitable for color balancing a substrate having a blue absorbing dye.
FIG. 22 shows the spectral transmittance. of a substrate having a blue absorbing dye.
FIG. 23 shows the color plot of a substrate having a blue absorbing dye.
FIG. 24 shows the spectral transmittance of a substrate having a blue absorbing dye and a rear AR coating.
FIG. 25 shows the color plot of a substrate having a blue absorbing dye and a rear AR coating.
FIG. 26 shows the spectral transmittance of a substrate having a blue absorbing dye and AR coatings on the front and rear surfaces.
FIG. 27 shows the color plot of a substrate having a blue absorbing dye and AR coatings on the front and rear surfaces.
FIG. 28 shows the spectral transmittance of a substrate having a blue absorbing dye and a color balancing AR coating.
FIG. 29 shows the color plot of a substrate having a blue absorbing dye and a color balancing AR coating.

### DETAILED DESCRIPTION

Embodiments of the present invention relate to an ophthalmic system that performs effective blue blocking while at the same time providing a cosmetically attractive product, normal or acceptable color perception for a user, and a high level of transmitted light for good visual acuity. An ophthalmic system is provided that can provide an average transmission of 80% or better transmission of visible light, inhibit selective wavelengths of blue light ("blue blocking"), allow for the wearer's proper color vision performance, and provide a mostly color neutral appearance to an observer looking at the wearer wearing such a lens or lens system. As used herein, the "average transmission" of a system refers to the average transmission at wavelengths in a range, such as the visible spectrum. A system also may be characterized by the "luminous transmission" of the system, which refers to an average in a wavelength range, that is weighted according to the sensitivity of the eye at each wavelength. Systems described herein may use various optical coatings, films, materials, and absorbing dyes to produce the desired effect.

More specifically, embodiments of the invention may provide effective blue blocking in combination with color balancing. "Color balancing" or "color balanced" as used herein means that the yellow or amber color, or other unwanted effect of blue blocking is reduced, offset, neutralized or otherwise compensated for so as to produce a cosmetically acceptable result, without at the same time reducing the effectiveness of the blue blocking. For example, wavelengths at or near 400 nm - 460 nm may be blocked or reduced in intensity. In particular, for example, wavelengths at or near 420 - 440 nm may be blocked or reduced in intensity. Furthermore, transmission of unblocked wavelengths may remain at a high level, for example at least 80%. Additionally, to an external viewer, the ophthalmic system may look clear or mostly clear. For a system user, color perception may be normal or acceptable.

An "ophthalmic system" as used here includes prescription or non-prescription ophthalmic lenses used, e.g., for glasses (or spectacles), sunglasses, contact lenses, intra-ocular lenses, corneal inlays, corneal on-lays, and may be treated or processed or combined with other components to provide desired functionalities described in further detail herein. As used herein, an "ophthalmic material" is one commonly used to fabricate an ophthalmic system, such as a corrective lens. Exemplary ophthalmic materials include glass, plastics such as CR-39, Trivex, and polycarbonate materials, though other materials may be used and are known for various ophthalmic systems.

An ophthalmic system may include a blue blocking component posterior to a color-balancing component. Either of the blue blocking component or the color balancing component may be, or form part of, an ophthalmic component such as a lens. The posterior blue blocking component and anterior color balancing component may be distinct layers on or adjacent to or near a surface or surfaces of an ophthalmic lens. The color-balancing component may reduce or neutralize a yellow or amber tint of the posterior blue blocking component, to produce a cosmetically acceptable appearance. For example, to an external viewer, the ophthalmic system may look clear or mostly clear. For a system user, color perception may be normal or acceptable. Further, because the blue blocking and color balancing tints are not intermixed, wavelengths in the blue light spectrum may be blocked or reduced in intensity and the transmitted intensity of incident light in the ophthalmic system may be at least 85% for unblocked wavelengths.

As discussed previously, techniques for blue blocking are known. The known techniques to block blue light wavelengths include absorption, reflection, interference, or any combination thereof. As discussed earlier, according to one technique, a lens may be tinted/dyed with a blue blocking tint, such as BPI Filter Vision 450 or BPI Diamond Dye 500, in a suitable proportion or concentration. The tinting may be accomplished, for example, by immersing the lens in a heated tint pot containing a blue blocking dye solution for some predetermined period of time. According to another technique, a filter is used for blue blocking. The filter could include, for example, organic or inorganic compounds exhibiting absorption and/or reflection of and/or interference with blue light wavelengths. The filter could comprise multiple thin layers or coatings of organic and/or inorganic substances. Each layer may have properties, which, either individually or in combination with other layers, absorbs, reflects or interferes with light having blue light wavelengths. Rugate notch filters are one example of blue blocking filters. Rugate filters are single thin films of inorganic dielectrics in which the refractive index oscillates continuously between high and low values. Fabricated by the co-deposition of two materials of different refractive index (e.g. SiO₂ and TiO₂), rugate filters are known to have very well defined stop-bands for wavelength blocking, with very little attenuation outside the band. The construction parameters of the filter (oscillation period, refractive index modulation, number of refractive index oscillations) determine the performance parameters of the filter (center of the stop-band, width of the stop band, transmission within the band). Rugate filters are disclosed in more detail in, for example, U.S. Patent Nos. 6,984,038 and 7,066,596, each of which is by reference in its entirety. Another technique for blue blocking is the use of multi-layer dielectric stacks. Multi-layer dielectric stacks are fabricated by depositing discrete layers of alternating high and low refractive index materials. Similarly to rugate filters, design parameters such as individual layer thickness, individual layer refractive index, and number of layer repetitions determine the performance parameters for multi-layer dielectric stacks.

Color balancing may comprise imparting, for example, a suitable proportion or concentration of blue tinting/dye, or a suitable combination of red and green tinting/dyes to the color-balancing component, such that when viewed by an external observer, the ophthalmic system as a whole has a cosmetically acceptable appearance. For example, the ophthalmic system as a whole may look clear or mostly clear.

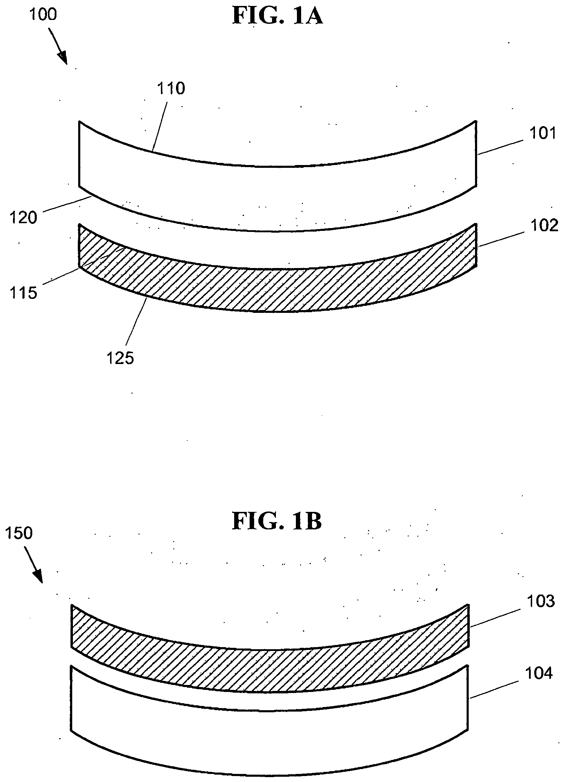

FIG. 1A shows an ophthalmic system including a posterior blue blocking component 101 and an anterior color-balancing component 102. Each component has a concave posterior side or surface 110, 115 and a convex anterior side or surface 120, 125. In system 100, the posterior blue blocking component 101 may be or include an ophthalmic component, such as a single vision lens, wafer or optical pre-form. The single vision lens, wafer or optical pre-form may be tinted or dyed to perform blue blocking. The anterior color-balancing component 102 may comprise a surface cast layer, applied to the single vision lens, wafer or optical pre-form according to known techniques. For example, the surface cast layer may be affixed or bonded to the single vision lens, wafer or optical pre-form using visible or UV light, or a combination of the two.

The surface cast layer may be formed on the convex side of the single vision lens, wafer or optical pre-form. Since the single vision lens, wafer or optical pre-form has been tinted or dyed to perform blue blocking, it may have a yellow or amber color that is undesirable cosmetically. Accordingly, the surface cast layer may, for example, be tinted with a suitable proportion of blue tinting/dye, or a suitable combination of red and green tinting/dyes.

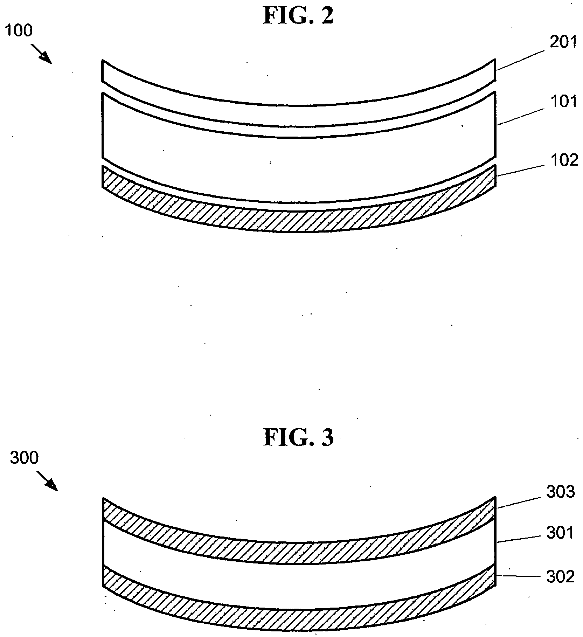

The surface cast layer may be treated with color balancing additives after it is applied to the single vision lens, wafer or optical pre-form that has already been processed to make it blue blocking. For example, the blue blocking single vision lens, wafer or optical pre-form with the surface cast layer on its convex surface may be immersed in a heated tint pot which has the appropriate proportions and concentrations of color balancing dyes in a solution. The surface cast layer will take up the color balancing dyes from the solution. To prevent the blue blocking single vision lens, wafer or optical pre-form from absorbing any of the color balancing dyes, its concave surface may be masked or sealed off with a dye resist, e.g. tape or wax or other coating. This is illustrated in FIG. 2, which shows an ophthalmic system 100 with a dye resist 201 on the concave surface of the single vision lens, wafer or optical pre-form 101. The edges of the single vision lens, wafer or optical pre-form may be left uncoated to allow them to become cosmetically color adjusted. This may be important for negative focal lenses having thick edges.

FIG. 1B shows another ophthalmic system 150 in which the anterior color-balancing component 104 may be or include an ophthalmic component, such as a single vision or multi-focal lens, wafer or optical pre-form. The posterior blue blocking component 103 may be a surface cast layer. To make this combination, the convex surface of the color balancing single vision lens, wafer or optical pre-form may be masked with a dye resist as described above, to prevent it taking up blue blocking dyes when the combination is immersed in a heated tint pot containing a blue blocking dye solution. Meanwhile, the exposed surface cast layer will take up the blue blocking dyes.

It should be understood that the surface cast layer could be used in combination with a multi-focal, rather than a single vision, lens, wafer or optical pre-form. In addition, the surface cast layer could be used to add power to a single vision lens, wafer or optical pre-form, including multi-focal power, thus converting the single vision lens, wafer or optical pre-form to a multi-focal lens, with either a lined or progressive type addition. Of course, the surface cast layer could also be designed to add little or no power to the single vision lens, wafer or optical pre-form.

FIG. 3 shows blue blocking and color balancing functionality integrated into an ophthalmic component. More specifically, in ophthalmic lens 300, a portion 303 corresponding to a depth of tint penetration into an otherwise clear or mostly clear ophthalmic component 301 at a posterior region thereof may be blue blocking. Further, a portion 302, corresponding to a depth of tint penetration into the otherwise clear or mostly clear ophthalmic component 301 at a frontal or anterior region thereof may be color balancing. The system illustrated in FIG. 3 may be produced as follows. The ophthalmic component 301 may, for example, initially be a clear or mostly clear single vision or multi-focal lens, wafer or optical pre-form. The clear or mostly clear single vision or multi-focal lens, wafer or optical pre-form may be tinted with a blue blocking tint while its front convex surface is rendered non-absorptive, e.g., by masking or coating with a dye resist as described previously. As a result, a portion 303, beginning at the posterior concave surface of the clear or mostly clear single vision or multi-focal lens, wafer or optical pre-form 301 and extending inwardly, and having blue blocking functionality, may be created by tint penetration. Then, the anti-absorbing coating of the front convex surface may be removed. An anti-absorbing coating may then be applied to the concave surface, and the front convex surface and peripheral edges of the single vision or multi-focal lens, wafer or optical pre-form may be tinted (e.g. by immersion in a heated tint pot) for color balancing. The color balancing dyes will be absorbed by the peripheral edges and a portion 302 beginning at the front convex surface and extending inwardly, that was left untinted due to the earlier coating. The order of the foregoing process could be reversed, i.e., the concave surface could first be masked while the remaining portion was tinted for color balancing. Then, the coating could be removed and a depth or thickness at the concave region left untinted by the masking could be tinted for blue blocking.

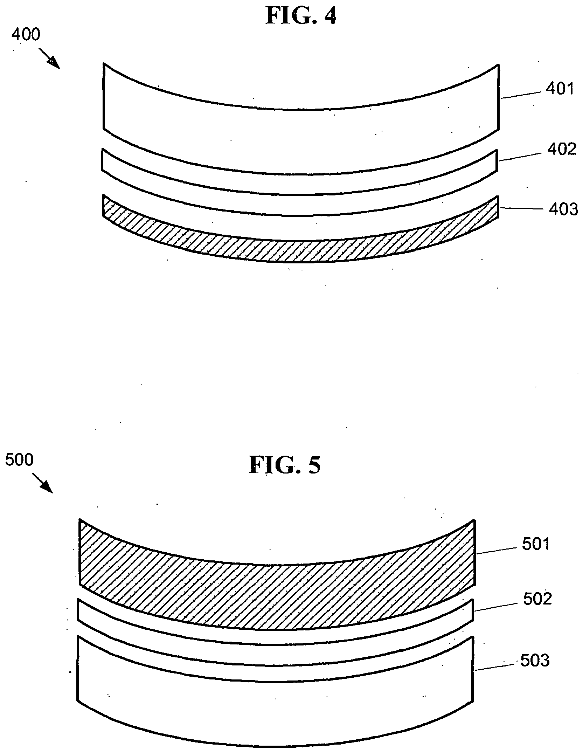

Referring now to FIG. 4, an ophthalmic system 400 may be formed using an in-mold coating. More specifically, an ophthalmic component 401 such as a single vision or multi-focal lens, wafer or optical pre-form which has been dyed/tinted with a suitable blue blocking tint, dye or other additive may be color balanced via surface casting using a tinted in-mold coating 403. The in-mold coating 403, comprising a suitable level and/or mixtures of color balancing dyes, may be applied to the convex surface mold (i.e., a mold, not shown, for applying the coating 403 to the convex surface of the ophthalmic component 401). A colorless monomer 402 may be filled in and cured between the coating 403 and ophthalmic component 401 The process of curing the monomer 402 will cause the color balancing in-mold coating to transfer itself to the convex surface of the ophthalmic component 401. The result is a blue blocking ophthalmic system with a color balancing surface coating. The in-mold coating could be, for example, an anti-reflective coating or a conventional hard coating.

Preferring now to FIG. 5, an ophthalmic system 500 may comprise two ophthalmic components, one blue blocking and the other color balancing. For example, a first ophthalmic component 501 could be a back single vision or concave surface multi-focal lens, wafer or optical pre-form, dyed/tinted with the appropriate blue blocking tint to achieve the desired level of blue blocking. A second ophthalmic component 503 could be a front single vision or convex surface multi-focal lens, wafer or optical pre-form, bonded or affixed to the back single vision or concave surface multi-focal lens, wafer or optical pre-form, for example using a UV or visible curable adhesive 502. The front single vision or convex surface multi-focal lens, wafer or optical pre-form could be rendered color balancing either before or after it was bonded with the back single vision or concave surface multi-focal lens, wafer or optical pre-form. If after, the front single vision or convex surface multi-focal lens, wafer or optical pre-form could be rendered color balancing, for example, by techniques described above. For example, the back single vision or concave surface multi-focal lens, wafer or optical pre-form may be masked or coated with a dye resist to prevent it taking up color balancing dyes. Then, the bonded back and front portions may be together placed in a heated tint pot containing a suitable solution of color balancing dyes, allowing the front portion to take up color balancing dyes.

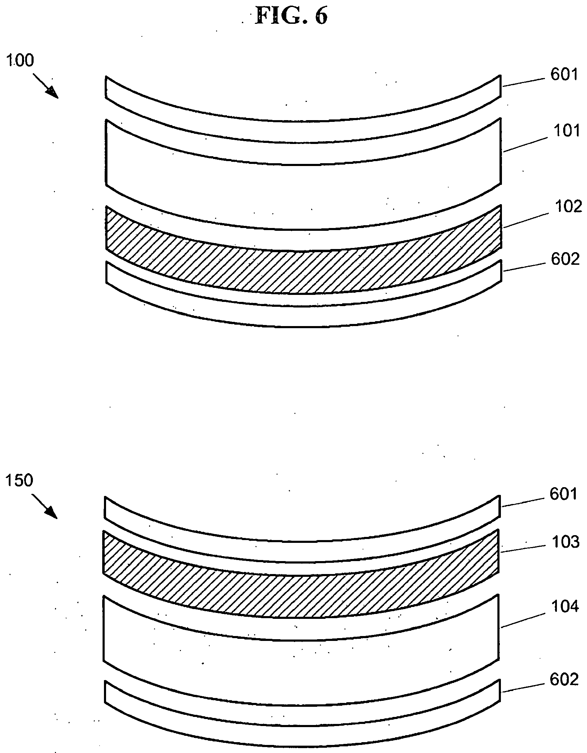

Any of the above-described embodiments systems, may be combined with one or more anti-reflective (AR) components. This is shown in FIG. 6, by way of example, for the ophthalmic lenses 100 and 150 shown in FIGs. 1A and 1B. In FIG. 6, a first AR component 601, e.g. a coating, is applied to the concave surface of posterior blue blocking element 101, and a second AR component 602 is applied to the convex surface of color balancing component 102. Similarly, a first AR component 601 is applied to the concave surface of posterior blue blocking component 103, and a second AR component 602 is applied to the convex surface of color balancing component 104.

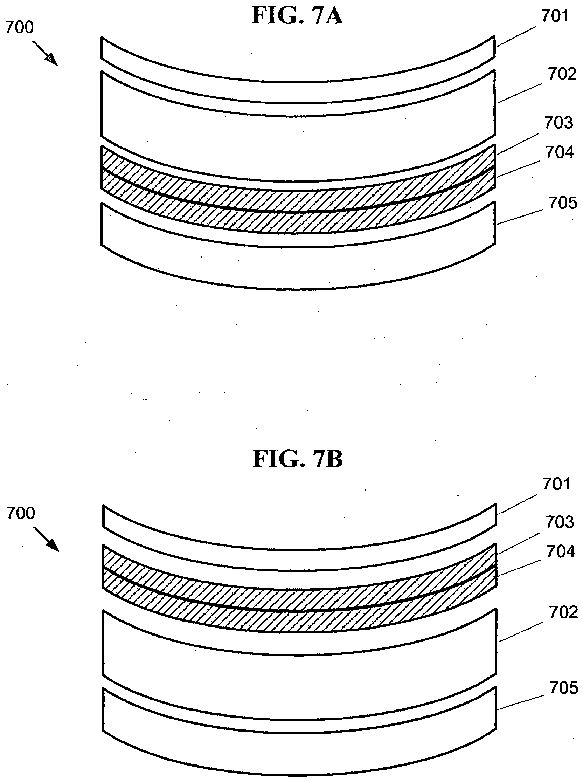

FIGS. 7A-7C show further exemplary systems including a blue blocking component and a color-balancing component. In FIG. 7A, an ophthalmic system 700 includes a blue blocking component 703 and a color balancing component 704 that are formed as adjacent, but distinct, coatings or layers on or adjacent to the anterior surface of a clear or mostly clear ophthalmic lens 702. The blue blocking component 703 is posterior to the color-balancing component 704. On or adjacent to the posterior surface of the clear or mostly clear ophthalmic lens, an AR coating or other layer 701 may be formed. Another AR coating or layer 705 may be formed on or adjacent to the anterior surface of the color-balancing layer 704.

In FIG. 7B, the blue blocking component 703 and color-balancing component 704 are arranged on or adjacent to the posterior surface of the clear or mostly clear ophthalmic lens 702. Again, the blue blocking component 703 is posterior to the color-balancing component 704. An AR component 701 may be formed on or adjacent to the posterior surface of the blue blocking component 703. Another AR component 705 may be formed on or adjacent to the anterior surface of the clear or mostly clear ophthalmic lens 702.

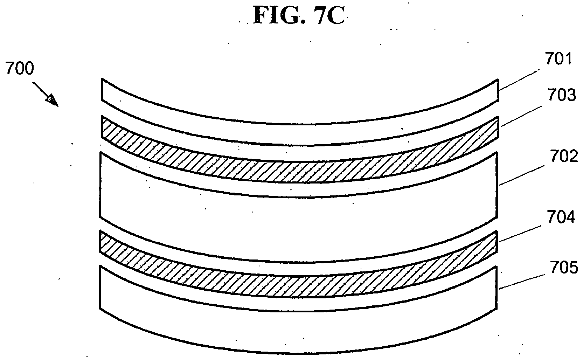

In FIG. 7C, the blue blocking component 703 and the color-balancing component 704 are arranged on or adjacent to the posterior and the anterior surfaces, respectively, of the clear ophthalmic lens 702. Again, the blue blocking component 703 is posterior to the color-balancing component 704. An AR component 701 may be formed on or adjacent to the posterior surface of the blue blocking component 703, and another AR component 705 may be formed on or adjacent to the anterior surface of the color-balancing component 704.

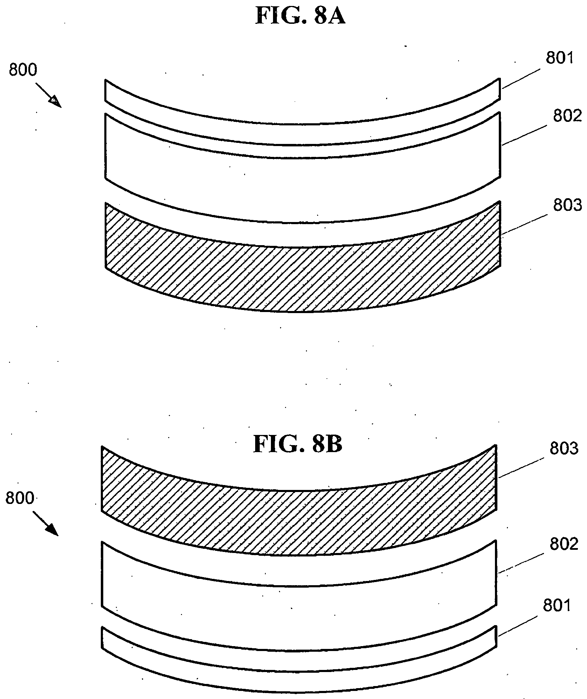

FIGs. 8A and 8B show an ophthalmic system 800 in which functionality to both block blue light wavelengths and to perform color balancing may be combined in a single component 803. For example, the combined functionality component may block blue light wavelengths and reflect some green and red wavelengths as well, thus neutralizing the blue and eliminating the appearance of a dominant color in the lens. The combined functionality component 803 may be arranged on or adjacent to either the anterior or the posterior surface of a clear ophthalmic lens 802. The ophthalmic lens 800 may further include an AR component 801 on or adjacent to either the anterior or the posterior surface of the clear ophthalmic lens 802.

To quantify the effectiveness of a color balancing component, it may be useful to observe light reflected and/or transmitted by a substrate of an ophthalmic material. The observed light may be characterized by its CIE coordinates to indicate the color of observed light; by comparing these coordinates to the CIE coordinates of the incident light, it is possible to determine how much the color of the light was shifted due to the reflection/transmission. White light is defined to have CIE coordinates of (0.33, 0.33). Thus, the closer an observed light's CIE coordinates are to (0.33, 0.33), the "more white" it will appear to an observer. To characterize the color shifting or balancing performed by a lens, (0.33, 0.33) white light may be directed at the lens, and the CIE of reflected and transmitted light observed. If the transmitted light has a CIE of about (0.33, 0.33), there will be no color shifting, and items viewed through the lens will have a natural appearance, i,e., the color will not be shifted relative to items observed without the lens. Similarly, if the reflected light has a CIE of about (0.33, 0.33), the lens will have a natural cosmetic appearance, i.e., it will not appear tinted to an observer viewing a user of the lens or ophthalmic system. Thus, it is desirable for transmitted and reflected light to have a CIE as close to (0.33, 0.33) as possible.

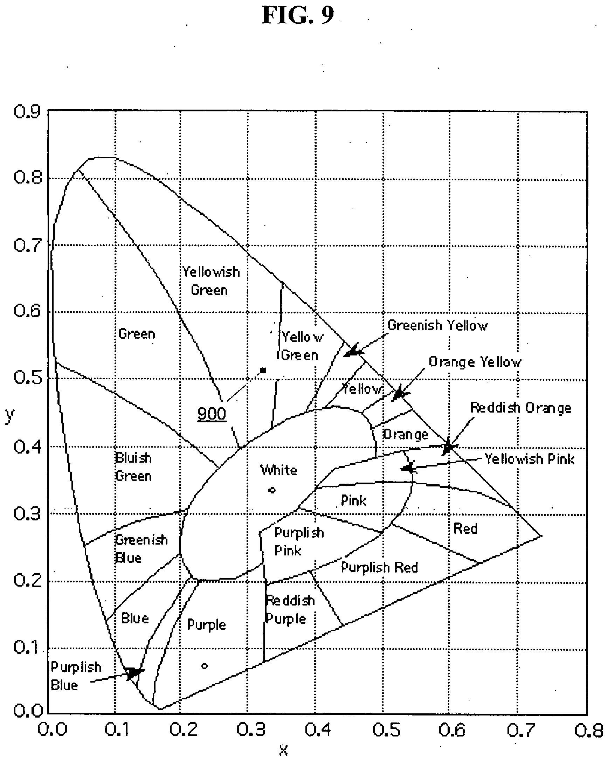

FIG. 9 shows a CIE plot indicating the observed colors corresponding to various CIE coordinates. A reference point 900 indicates the coordinates (0.33, 0.33). Although the central region of the plot typically is designated as "white," some light having CIE coordinates in this region can appear slightly tinted to a viewer. For example, light having CIE coordinates of (0.4, 0.4) will appear yellow to an observer. Thus, to achieve a color-neutral appearance in an ophthalmic system, it is desirable for (0.33, 0.33) light (i.e., white light) that is transmitted and/or reflected by the system to have CIE coordinates as close to (0.33, 0.33) as possible after the transmission/reflection. The CIE plot shown in FIG. 9 will be used herein as a reference to show the color shifts observed with various systems, though the labeled regions will be omitted for clarity.

Absorbing dyes may be included in the substrate material of an ophthalmic lens by injection molding the dye into the substrate material to produce lenses with specific light transmission and absorption properties. These dye materials can absorb at the fundamental peak wavelength of the dye or at shorter resonance wavelengths due to the presence of a Soret band typically found in porphyrin materials. Exemplary ophthalmic materials include various glasses and polymers such as CR-39^{®}, TRIVEX^{®}, polycarbonate, polymethylmethacrylate, silicone, and fluoropolymers, though other materials may be used and are known for various ophthalmic systems.

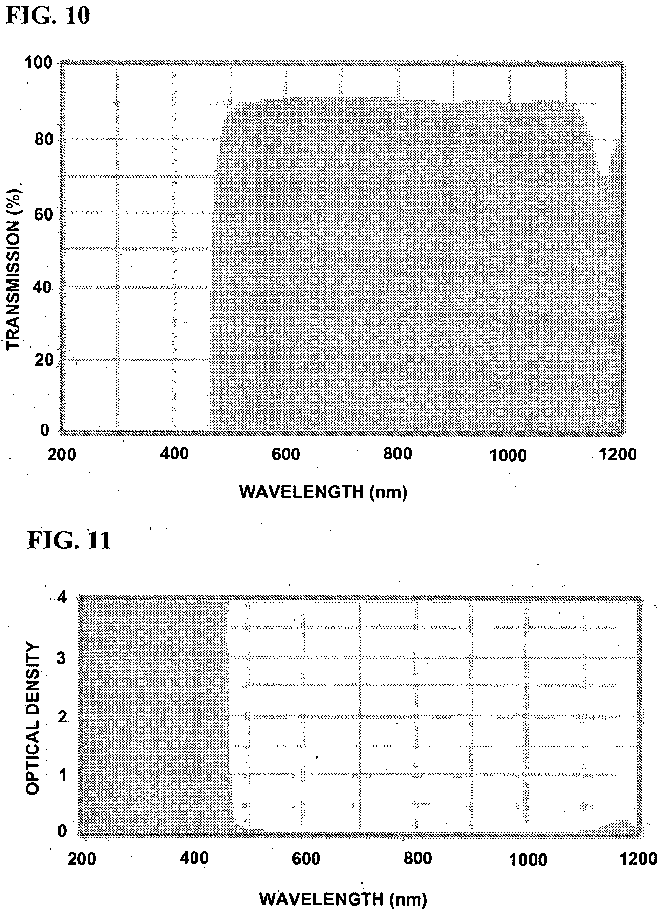

By way of example only, Gentex day material E465 transmittance and absorbance is shown in FIGS. 10-11. The Absorbance (A) is related to the transmittance (T) by the equation, A = log₁₀(1/T). In this case, the transmittance is between 0 and 1 (0 < T < 1). Often transmittance is express as a percentage, i.e., 0% < T < 100%. The E465 dye blocks those wavelengths less than 465 and is normally provided to block these wavelengths with high optical density (OD > 4). Similar products are available to block other wavelengths. For example, E420 from Gentex blocks wavelengths below 420nm. Other exemplary dyes include porphyrins, perylene, and similar dyes that can absorb at blue wavelengths.

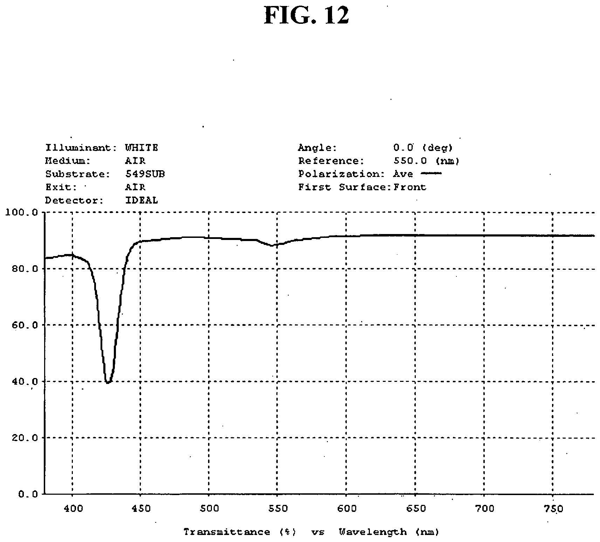

The absorbance at shorter wavelengths can be reduced by a reduction of the dye concentration. This and other dye materials can achieve a transmittance of∼50% in the 430nm region. FIG. 12 shows the transmittance of a polycarbonate substrate with a dye concentration suitable for absorbing in the 430nm range, and with some absorption in the range of 420nm - 440nm. This was achieved by reducing the concentration of the dye and including the effects of a polycarbonate substrate. The rear surface is at this point not antireflection coated.

The concentration of dye also may affect the appearance and color shift of an ophthalmic system. By reducing the concentration, systems with varying degrees of color shift may be obtained. A "color shift" as used herein refers to the amount by which the CIE coordinates of a reference light change after transmission and/or reflection of the ophthalmic system. It also may be useful to characterize a system by the color shift causes by the system due to the differences in various types of light typically perceived as white (e.g., sunlight, incandescent light, and fluorescent light). It therefore may be useful to characterize a system based on the amount by which the CIE coordinates of incident light are shifted when the light is transmitted and/or reflected by the system. For example, a system in which light with CIE coordinates of (0.33, 0.33) becomes light with a CIE of (0.30, 0.30) after transmission may be described as causing a color shift of (-.03, -.03), or, more generally, (±0.03, ±0,03). Thus the color shift caused by a system indicates how "natural" light and viewed items appear to a wearer of the system. As further described below, systems causing color shifts of less than (±0.05, ±0.05) to (±0.02, ±0.02) have been achieved.

A reduction in short-wavelength transmission in an ophthalmic system may be useful in reducing cell death due to photoelectric effects in the eye, such as excitation of A2E. It has been shown that reducing incident.light at 430±30 nm by about 50% can reduce cell death by about 80%. See, for example, Janet R. Sparrow et al., "Blue light-absorbing intraocular lens and retinal pigment epithelium protection in vitro," J. Cataract Refract. Surg. 2004, vol. 30, pp. 873-78. It is further believed that reducing the amount of blue light, such as light in the 430-460 nm range, by as little as 5% may similarly reduce cell death and/or degeneration, and therefore prevent or reduce the adverse effects of conditions such as atrophic age-related macular degeneration.

Although an absorbing dye may be used to block undesirable wavelengths of light, the dye may produce a color tint in the lens as a side effect. For example, many blue-blocking ophthalmic lenses have a yellow coloring that is often undesirable and/or aesthetically displeasing. To offset this coloring, a color balancing coating may be applied to one or both surfaces of a substrate including the absorbing dye therein.

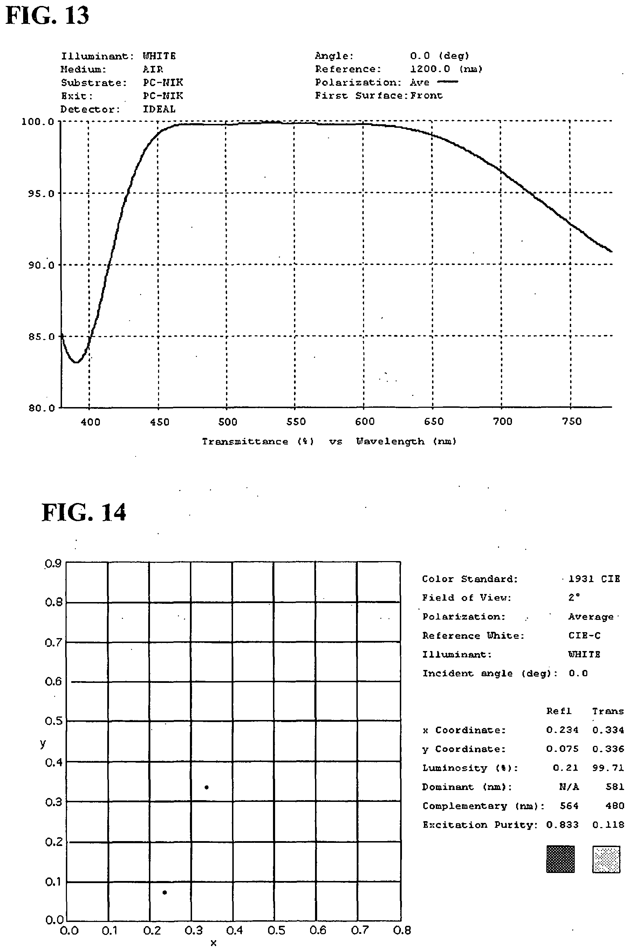

Antireflection (AR) coatings (which are interference filters) are well-established within the commercial ophthalmic coating industry. The coatings typically are a few layers, often less than 10, and typically are used to reduce the reflection from the polycarbonate surface to less than 1%. An example of such a coating on a polycarbonate surface is shown in FIG. 13. The color plot of this coating is shown in FIG. 14 and it is observed that the color is quite neutral. The total reflectance was observed to be 0.21%. The reflected light was observed to have CIE coordinates of (0.234, 0.075); the transmitted light had CIE coordinates of (0.334, 0.336).

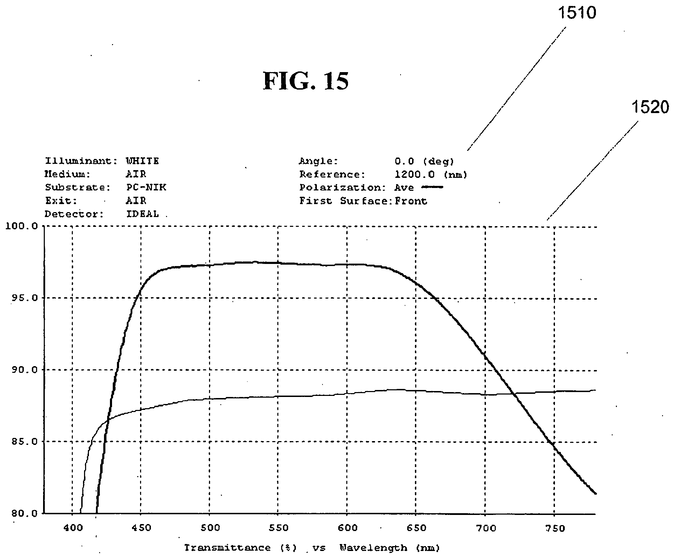

AR coatings may be applied to both surfaces of a lens or other ophthalmic device to achieve a higher transmittance. Such a configuration is shown in FIG. 15 where the darker line 1510 is the AR coated polycarbonate and the thinner line 1520 is an uncoated polycarbonate substrate. This AR coating provides a 10% increase in total transmitted light. There is some natural loss of light due to absorption in the polycarbonate substrate. The particular polycarbonate substrate used for this example has a transmittance loss of approximately 3%. In the ophthalmic industry AR coatings generally are applied to both surfaces to increase the transmittance of the lens.

In systems according to the present invention, AR coatings or other color balancing films may be combined with an absorbing dye to allow for simultaneous absorption of blue wavelength light, typically in the 430 nm region, and increased transmittance. As previously described, elimination of the light in the 430 nm region alone typically results in a lens that has some residual color cast. To spectrally tailor the light to achieve a color neutral transmittance, at least one of the AR coatings may be modified to adjust the overall transmitted color of the light. In ophthalmic systems according to the invention, this adjustment may be performed on the front surface of the lens to create the following lens structure:

Air (farthest from the user's eye) / Front convex lens coating / Absorbing ophthalmic lens substrate / rear concave anti-reflection coating /Air (closest to the user's eye).

In such a configuration, the front coating may provide spectral tailoring to offset the color cast resulting from the absorption in the substrate in addition to the antireflective function typically performed in conventional lenses. The lens therefore may provide an appropriate color balance for both transmitted and reflected light. In the case of transmitted light the color balance allows for proper color vision; in the case reflected light the color balance may provide the appropriate lens aesthetics.

In some cases, a color balancing film may be disposed between two layers of other ophthalmic material. For example, a filter, AR film, or other film may be disposed within an opthalmic material. For example, the following configuration may be used:

Air (farthest from the user's eye) / ophthalmic material / film / ophthalmic material / air (closest to user's eye).

The color balancing film also may be a coating, such as a hardcoat, applied to the outer and/or inner surface of a lens. Other configurations are possible. For example, referring to FIG. 3, an ophthalmic system may include an ophthalmic material 301 doped with a blue-absorbing dye and one or more color balancing layers 302, 303. In another configuration, an inner layer 301 may be a color balancing layer surrounded by ophthalmic material 302, 303 doped with a blue-absorbing dye. Additional layers and/or coatings, such as AR coatings, may be disposed on one or more surfaces of the system. It will be understood how similar materials and configurations may be used, for example in the systems described with respect to FIGS. 4-8B.

Thus, optical films and/or coatings such as AR coatings may be used to fine-tune the overall spectral response of a lens having an absorbing dye. Transmission variation across the visible spectrum is well known and varies as a function of the thickness and number of layers in the optical coating. In the invention one or more layers can be used to provide the needed adjustment of the spectral properties.

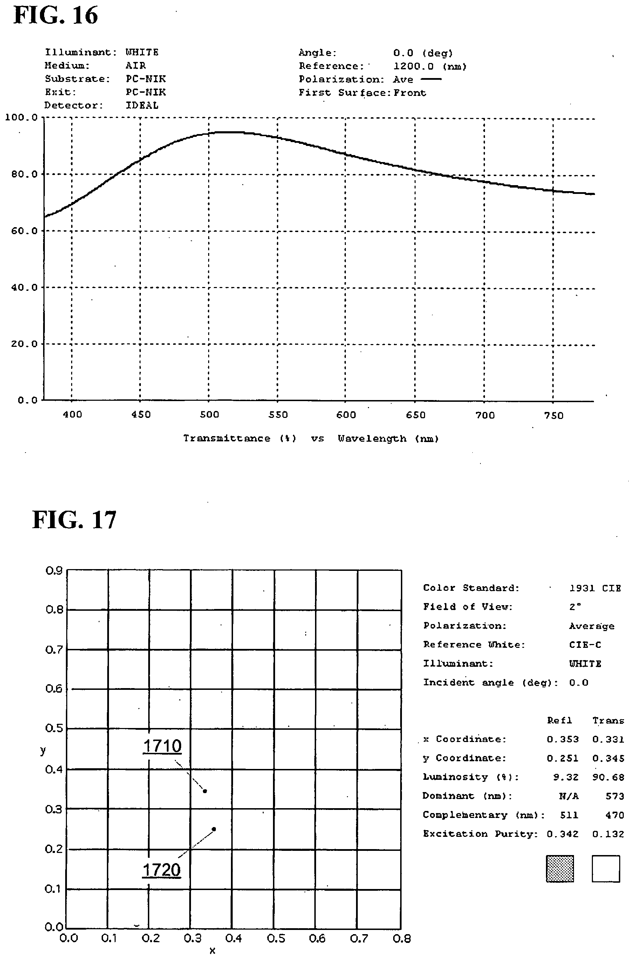

In an exemplary system, color variation is produced by a single layer of TiO₂ (a common AR coating material). Fig. 16 shows the spectral transmittance of a 106nm thick single layer of TiO₂. The color plot of this same layer is shown in FIG. 17. The CIE color coordinates (x, y) 1710 shown for the transmitted light are (0.331, 0.345). The reflected light had CIE coordinates of (0.353, 0.251) 1720, resulting in a purplish-pink color.

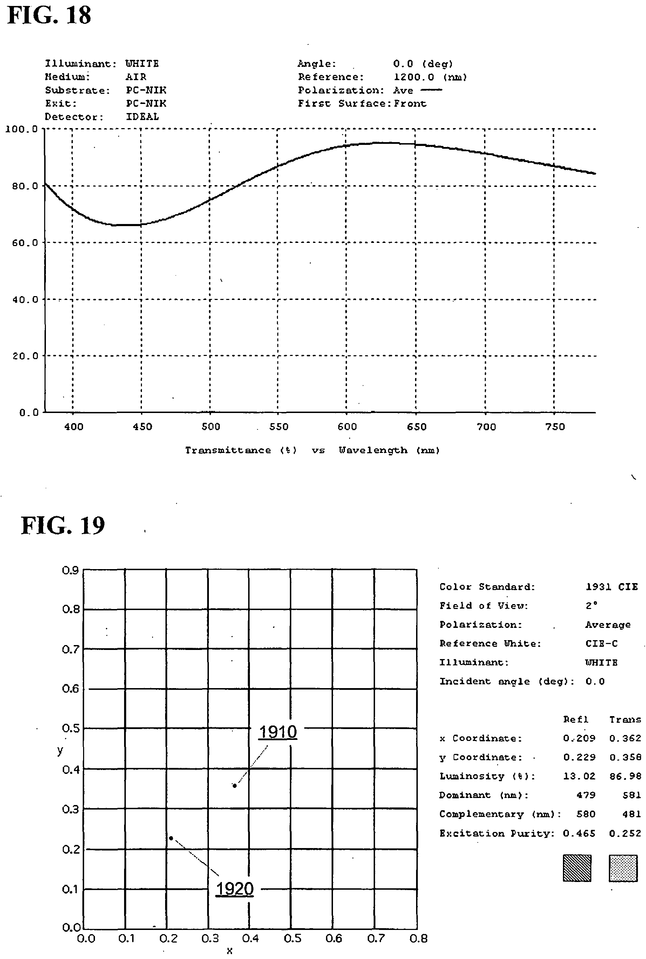

Changing the thickness of the TiO₂ layer changes the color of the transmitted light as shown in the transmitted spectra and color plot for a 134 nm layer, shown in FIGS. 18 and 19 respectively. In this system, the transmitted light exhibited CIE coordinates of (0.362, 0.368) 1910, and the reflected light had CIE coordinates of (0.209, 0.229) 1920. The transmission properties of various AR coatings and the prediction or estimation thereof are known in the art. For example, the transmission effects of an AR coating formed of a known thickness of an AR material may be calculated and predicted using various computer programs. Exemplary, nonlimiting programs include Essential Macleod Thin Films Software available from Thin Film Center, Inc., TFCalc available from Software Spectra, Inc., and FilmStar Optical Thin Film Software available from FTG Software Associates. Other methods may be used to predict the behavior of an AR coating or other similar coating or film.

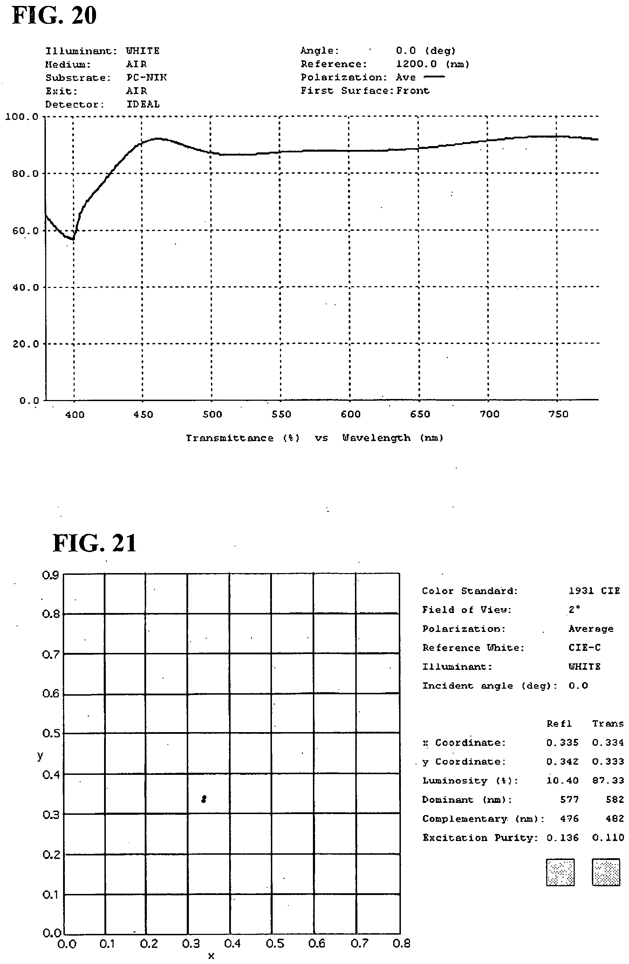

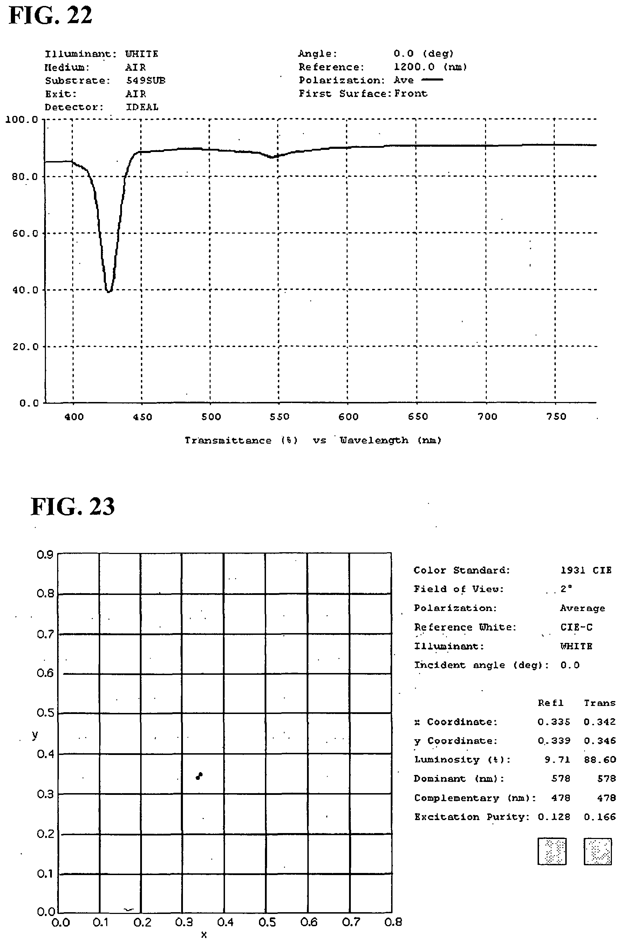

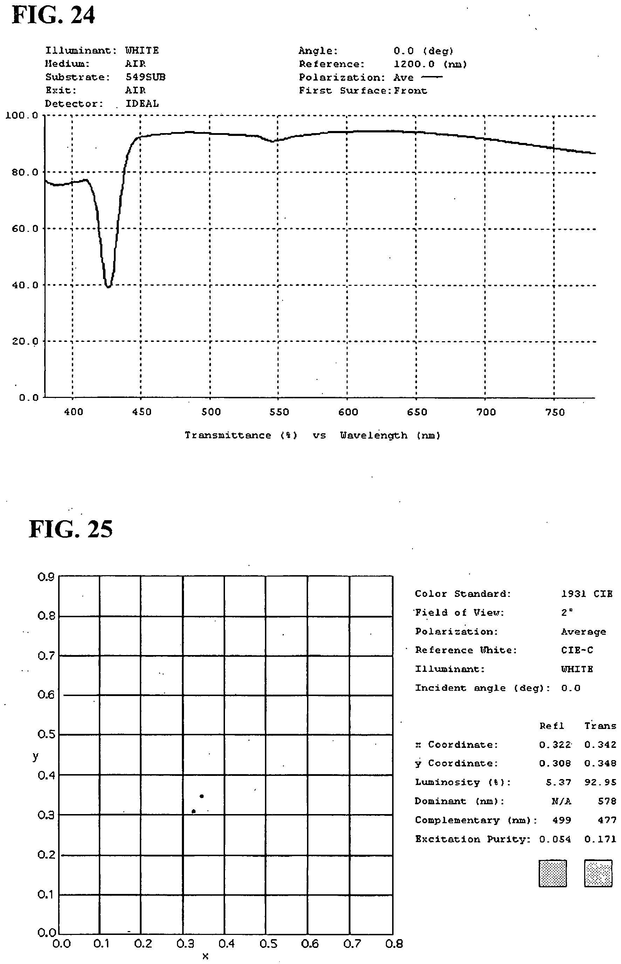

In systems according to the present invention, a blue-absorbing dye may be combined with a coating or other film to provide a blue blocking, color balanced system. The coating may be an AR coating on the front surface that is modified to correct the color of the transmitted and/or reflected light. The transmittance and color plot of an exemplary AR coating are shown in FIGS. 20 and 21, respectively. FIGS. 22 and 23 show the transmittance and color plot, respectively, for a polycarbonate substrate having a blue absorbing dye without an AR coating. The dyed substrate absorbs most strongly in the 430 nm region, including some absorption in the 420 - 440 nm region. The dyed substrate may be combined with an appropriate AR coating as illustrated in FIGS. 20-21 to increase the overall transmittance of the system. The transmittance and color plot for a dyed substrate having a rear AR coating are shown in FIGS. 24 and 25, respectively.

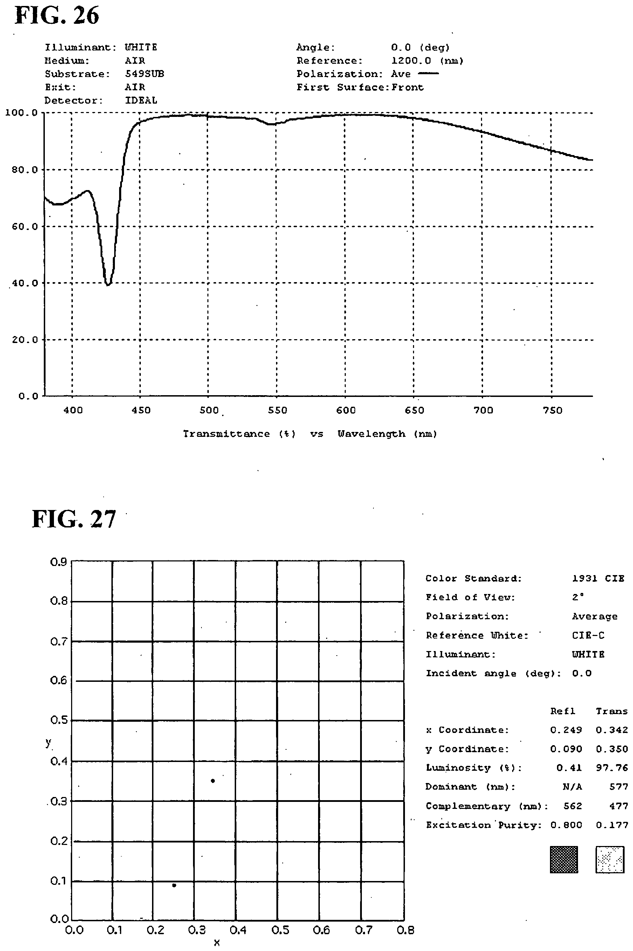

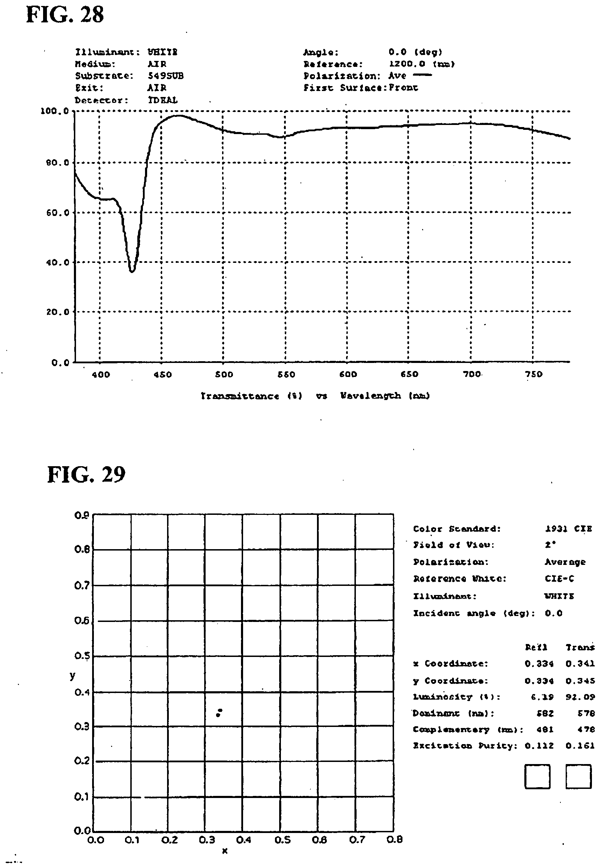

An AR coating also may be applied to the front of an ophthalmic system (i.e., the surface farthest from the eye of a wearer of the system), resulting in the transmittance and color plot shown in FIGS. 26 and 27, respectively. Although the system exhibits a high transmission and transmitted light is relatively neutral, the reflected light has a CIE of (0.249, 0.090). Therefore, to more completely color balance the effects of the blue absorbing dye, the front AR coating may be modified to achieve the necessary color balance to produce a color neutral configuration. The transmittance and the color plot of this configuration are shown in FIGS. 28 and 29 respectively. In this configuration, both the transmitted and reflected light may be optimized to achieve color neutrality. It may be preferred for the interior reflected light to be about 6%. Should the reflectivity level be annoying to the wearer of the system, the reflection can be further reduced by way of adding an additional different absorbing dye into the lens substrate that would absorb a different wavelength of visible light. However, the design of this configuration achieves remarkable performance and satisfies the need for a blue blocking, color balanced ophthalmic system as described herein. The total transmittance is over 90% and both the transmitted and reflected colors are quite close to the color neutral white point. As shown in FIG. 27, the reflected light has a CIE of (0.334, 0.334), and the transmitted light has a CIE of (0.341, 0.345), indicating little or no color shifting.

In some configurations, the front modified anti-reflection coating can be designed to block 100% of the blue light wave length to be inhibited. However, this may result in a back reflection of about 9% to 10% for the wearer. This level of reflectivity can be annoying to the wearer. Thus by combining an absorbing dye into the lens substrate this reflection with the front modified anti-reflection coating the desired effect can be achieved along with a reduction of the reflectivity to a level that is well accepted by the wearer. The reflected light observed by a wearer of a system including one or more AR coatings may be reduced to 8% or less, or more preferably 3% or less.

The combination of a front and rear AR coating may be referred to as a dielectric stack, and various materials and thicknesses may be used to further alter the transmissive and reflective characteristics of an ophthalmic system. For example, the front AR coating and/or the rear AR coating may be made of different thicknesses and/or materials to achieve a particular color balancing effect. In some cases, the materials used to create the dielectric stack may not be materials traditionally used to create antireflective coatings. That is, the color balancing coatings may correct the color shift caused by a blue absorbing dye in the substrate without performing an antireflective function.

As discussed previously, filters are another technique for blue blocking. Accordingly, any of the blue blocking components discussed could be or include or be combined with blue blocking filters. Such filters may include rugate filters, interference filters, band-pass filters, band-block filters, notch filters or dichroic filters.

In embodiments of the invention, one or more of the above-disclosed blue-blocking techniques may be used in conjunction with other blue-blocking techniques. By way of example only, a lens or lens component may utilize both a dye/tint and a rugate notch filter to effectively block blue light.

Any of the above-disclosed structures and techniques may be employed in an ophthalmic system according to the present invention to perform blocking of blue light wavelengths at or near 400-460 nm. For example, in embodiments the wavelengths of blue light blocked may be within a predetermined range. In embodiments, the range may be 430 nm ± 30 nm. In other embodiments, the range may be 430 nm ± 20 nm. In still other embodiments, the range may be 430 nm ± 10 nm. In embodiments, the ophthalmic system may limit transmission of blue wavelengths within the above-defined ranges to substantially 90% of incident wavelengths. In other embodiments, the ophthalmic system may limit transmission of the blue wavelengths within the above-defined ranges to substantially 80% of incident wavelengths. In other embodiments, the ophthalmic system may limit transmission of the blue wavelengths within the above-defined ranges to substantially 70% of incident wavelengths. In other embodiments, the ophthalmic system may limit transmission of the blue wavelengths within the above-defined ranges to substantially 60% of incident wavelengths. In other embodiments, the ophthalmic system may limit transmission of the blue wavelengths within the above-defined ranges to substantially 50% of incident wavelengths. In other embodiments, the ophthalmic system may limit transmission of the blue wavelengths within the above-defined ranges to substantially 40% of incident wavelengths. In still other embodiments, the ophthalmic system may limit transmission of the blue wavelengths within the above-defined ranges to substantially 30% of incident wavelengths. In still other embodiments, the ophthalmic system may limit transmission of the blue wavelengths within the above-defined ranges to substantially 20% of incident wavelengths. In still other embodiments, the ophthalmic system may limit transmission of the blue wavelengths within the above-defined ranges to substantially 10% of incident wavelengths. In still other embodiments, the ophthalmic system may limit transmission of the blue wavelengths within the above-defined ranges to substantially 5% of incident wavelengths. In still other embodiments, the ophthalmic system may limit transmission of the blue wavelengths within the above-defined ranges to substantially 1% of incident wavelengths. In still other embodiments, the ophthalmic system may limit transmission of the blue wavelengths within the above-defined ranges to substantially 0% of incident wavelengths. Stated otherwise, attenuation by the ophthalmic system of the electromagnetic spectrum at wavelengths in the above-specified ranges may be at least 10%; or at least 20%; or at least 30%; or at least 40%; or at least 50%; or at least 60%; or at least 70%; or at least 80%; or at least 90%; or at least 95%; or at least 99%; or substantially 100%.

In some cases it may be particularly desirable to filter a relatively small portion of the blue spectrum, such as the 400 nm - 460 nm region. For example, it has been found that blocking too much of the blue spectrum can interfere with scotopic vision and circadian rhythms. Conventional blue blocking ophthalmic lenses typically block a much larger amount of a wide range of the blue spectrum, which can adversely affect the wearer's "biological clock" and have other adverse effects. Thus, it may be desirable to block a relatively narrow range of the blue spectrum as described herein. Exemplary systems that may filter a relatively small amount of light in a relatively small range include system that block or absorb 5-50%, 5-20%, and 5-10% of light having a wavelength of 400 nm - 460 nm, 410 nm - 450 nm, and 420 nm - 440 nm.

At the same time as wavelengths of blue light are selectively blocked as described above, at least 80%, or at least 85%, and in other embodiments at least 90-95%, of other portions of the visual electromagnetic spectrum may be transmitted by the ophthalmic system. Stated otherwise, attenuation by the ophthalmic system of the electromagnetic spectrum at wavelengths outside the blue light spectrum, e.g. wavelengths other than those in a range around 430 nm may be 20% or less, 15% or less, 10% or less, and in other embodiments, 5% or less.

Additionally, embodiments of the present invention may further block ultra-violet radiation the UVA and UVB spectral bands as well as infra-red radiation with wavelengths greater than 700 nm.

Any of the above-disclosed ophthalmic system may be incorporated into an article of eyewear, including externally-worn eyewear such as eyeglasses, sunglasses, goggles or contact lenses. In such eyewear, because the blue-blocking component of the systems is posterior to the color balancing component, the blue-blocking component will always be closer to the eye than the color-balancing component when the eyewear is worn. The ophthalmic system may also be used in such articles of manufacture as surgically implantable intra-ocular lenses.

Several embodiments of the invention are specifically illustrated 'and/or described herein. However, it will be appreciated that obvious modifications and variations of the invention are covered by the above teachings and within the purview of the appended claims. The present invention is defined by the features of the claims.

## Claims

1. An ophthalmic system (100, 200, 300, 400, 500) comprising:
a first layer comprising:
an ophthalmic material; and
a dye that selectively absorbs at least 5% of light in a wavelength range within 400 nm to 460 nm; and
a film disposed on the first layer;
wherein the film compensates for a colour imbalance resulting from absorption of light by the dye;
wherein the system (100, 200, 300, 400, 500) has an average transmission of at least 80% across the visible spectrum; and
wherein white light has CIE (x,y) coordinates of (0.33±0.05, 0.33±0.05), preferably (0.33±0.02, 0.33±0.02), after transmission through the ophthalmic system.

2. The ophthalmic system (100, 200, 300, 400, 500) of claim 1, further comprising a second layer of the ophthalmic material disposed on the film, wherein the film is disposed between the first layer and the second layer.

3. The ophthalmic system (100, 200, 300, 400, 500) of claim 1 wherein the film is an antireflective coating disposed on the outer surface of the ophthalmic substrate.

4. The ophthalmic system (100, 200, 300, 400, 500) of claim 1 further comprising an antireflective coating disposed on a surface of the system closest to the eye of a wearer of the system.

5. The ophthalmic system (100, 200, 300, 400, 500) of claim 4 wherein the reflected light observed by a wearer of the system is less than 8%, preferably less than 3%.

6. The ophthalmic system (100, 200, 300, 400, 500) of claim 1 wherein the dye is perylene, a porphyrin, or a similar dye that can absorb blue wavelengths.

7. The ophthalmic system (100, 200, 300, 400, 500) of claim 1, wherein said dye selectively absorbs at least 5% of light in a wavelength range within 400 nm-460 nm, and wherein the system is adapted to have an average transmission of at least 80% across the visible spectrum.

8. The ophthalmic system (100, 200, 300, 400, 500) of claim 7 wherein the system is adapted to have an average transmission of at least 90% across the visible spectrum.

9. A method of making an ophthalmic device, comprising:
providing a first layer comprising an ophthalmic material and a dye that selectively absorbs at least 5% of light in a wavelength range within 400 nm-460 nm;
and
depositing a film on the first layer;
wherein the film compensates for a colour imbalance resulting from absorption of light by the dye such that white light has CIE (x,y) coordinates of (0.33±0.05, 0.33±0.05), preferably (0.33±0.02, 0.33±0.02), after transmission through the device.

10. The method of claim 9 wherein the wavelength range is at or near 420 nm-440 nm.

11. The method of claim 10 wherein the wavelength range absorbed by the dye includes the absorption of at least 20%, preferably at least 50% of light having a wavelength of 430 nm.

12. The method of claim 9 wherein the film is an antireflective coating.

13. The method of claim 9, further comprising:
depositing a second layer of the ophthalmic material on the film, wherein the film is disposed between the first layer and the second layer.

14. The ophthalmic system of claim 7, wherein the wavelength range spans 60 nm, 40 nm, or 20 nm.

15. The ophthalmic system of claim 7, wherein the full width at half minimum of the selectively absorbed wavelength range is about 15 nm.

16. The ophthalmic system of claim 1 or 7 wherein the wavelength range is at or near 420 nm - 440 nm.

17. The ophthalmic system of claim 16 wherein the wavelength range absorbed by the dye includes the absorption of at least 20%, preferably at least 50%, of light having a wavelength of 430 nm.

## Patentansprüche

1. Ophthalmisches System (100, 200, 300, 400, 500), das aufweist:
eine erste Schicht, die aufweist:
ein ophthalmisches Material; und
einen Farbstoff, der selektiv mindestens 5% Licht in einem Wellenlängenbereich innerhalb von 400 nm bis 460 nm absorbiert; und
einen auf der ersten Schicht angeordneten Film;
wobei der Film ein Farbungleichgewicht ausgleicht, das von einer Lichtabsorption durch den Farbstoff herrührt;
wobei das System (100, 200, 300, 400, 500) eine durchschnittliche Transmission von mindestens 80% über das sichtbare Spektrum aufweist; und
wobei weißes Licht nach der Transmission durch das ophthalmische System CIE (x,y)-Koordinaten von (0,33±0,05, 0,33±0,05), vorzugsweise (0,33±0,02, 0,33±0,02) aufweist.

2. Ophthalmisches System (100, 200, 300, 400, 500) nach Anspruch 1, das ferner eine zweite Schicht des ophthalmischen Materials aufweist, die auf dem Film angeordnet ist, wobei der Film zwischen der ersten Schicht und der zweiten Schicht angeordnet ist.

3. Ophthalmisches System (100, 200, 300, 400, 500) nach Anspruch 1, wobei der Film eine Antireflexbeschichtung ist, die auf der Außenseite des ophthalmischen Substrats angeordnet ist.

4. Ophthalmisches System (100, 200, 300; 400, 500) nach Anspruch 1, das ferner eine Antireflexbeschichtung aufweist, die auf einer Oberfläche des Systems angeordnet ist, die dem Auge eines Trägers des Systems am nächsten liegt.

5. Ophthalmisches System (100, 200, 300, 400, 500) nach Anspruch 4, wobei das reflektierte Licht, das durch einen Träger des Systems beobachtet wird, weniger als 8%, vorzugsweise weniger als 3% beträgt.

6. Ophthalmisches System (100, 200, 300, 400, 500) nach Anspruch 1, wobei der Farbstoff Perylen, ein Porphyrin oder ein ähnlicher Farbstoff ist, der blaue Wellenlängen absorbieren kann.

7. Ophthalmisches System (100, 200, 300, 400, 500) nach Anspruch 1, wobei der Farbstoff selektiv mindestens 5% Licht in einem Wellenlängenbereich innerhalb von 400 nm bis 460 nm absorbiert, und wobei das System eingerichtet ist, eine durchschnittliche Transmission von mindestens 80% über das sichtbare Spektrum aufzuweisen.

8. Ophthalmisches System (100, 200, 300, 400, 500) nach Anspruch 7, wobei das System eingerichtet ist, eine durchschnittliche Transmission von mindestens 90% über das sichtbare Spektrum aufzuweisen.

9. Verfahren zum Herstellen einer ophthalmischen Vorrichtung, das aufweist:
Bereitstellen einer ersten Schicht, die ein ophthalmisches Material und einen Farbstoff aufweist, der selektiv mindestens 5% Licht in einem Wellenlängenbereich innerhalb von 400 nm bis 460 nm absorbiert; und
Abscheiden eines Films auf der ersten Schicht;
wobei der Film ein Farbungleichgewicht ausgleicht, das von einer Lichtabsorption durch den Farbstoff herrührt, so dass nach der Transmission durch die Vorrichtung weißes Licht CIE (x,y)-Koordinaten von (0,33±0,05, 0,33±0,05), vorzugsweise (0,33±0,02, 0,33±0,02), aufweist.

10. Verfahren nach Anspruch 9, wobei sich der Wellenlängenbereich bei oder nahe bei 420 nm bis 440 nm befindet.

11. Verfahren nach Anspruch 10, wobei der durch den Farbstoff absorbierte Wellenlängenbereich die Absorption von mindestens 20%, vorzugsweise mindestens 50% des Lichts umfasst, das eine Wellenlänge von 430 nm aufweist.

12. Verfahren nach Anspruch 9, wobei der Film eine Antireflexbeschichtung ist.

13. Verfahren nach Anspruch 9 das ferner aufweist:
Abscheiden einer zweiten Schicht des ophthalmischen Materials auf dem Film, wobei der Film zwischen der ersten Schicht und der zweiten Schicht angeordnet wird.

14. Ophthalmisches System nach Anspruch 7, wobei der Wellenlängenbereich 60 nm, 40 nm oder 20 nm überspannt.

15. Ophthalmisches System nach Anspruch 7, wobei die Halbwertsbreite des selektiv absorbierten Wellenlängenbereichs etwa 15 nm beträgt.

16. Ophthalmisches System nach Anspruch 1 oder 7, wobei sich der Wellenlängenbereich bei oder nahe bei 420 nm bis 440 nm befindet.

17. Ophthalmisches System nach Anspruch 16, wobei der durch den Farbstoff absorbierte Wellenlängenbereich die Absorption von mindestens 20%, vorzugsweise mindestens 50% des Lichts umfasst, das eine Wellenlänge von 430 nm aufweist.

## Revendications

1. Système ophtalmique (100, 200, 300, 400, 500) comprenant :
une première couche comportant :
un matériau ophtalmique ; et
un colorant absorbant sélectivement au moins 5 % de la lumière dans une plage de longueur d'onde comprise entre 400 nm et 460 nm ; et
un film disposé sur la première couche ;
où le film compense un déséquilibre de couleurs résultant de l'absorption de lumière par le colorant ;
où ledit système (100, 200, 300, 400, 500) présente une transmission moyenne d'au moins 80 % dans le spectre visible ; et
où la lumière blanche présente des coordonnées CIE (x, y) de (0,33±0,05, 0,33±0,05),
préférentiellement de (0,33±0,02, 0,33±0,02), après transmission par le système ophtalmique.

2. Système ophtalmique (100, 200, 300, 400, 500) selon la revendication 1, comprenant en outre une deuxième couche du matériau ophtalmique disposée sur le film, le film étant intercalé entre la première couche et la deuxième couche.

3. Système ophtalmique (100, 200, 300, 400, 500) selon la revendication 1, où le film est un revêtement anti-réfléchissant disposé sur la surface extérieur du substrat ophtalmique.

4. Système ophtalmique (100, 200, 300, 400, 500) selon la revendication 1, comprenant en outre un revêtement anti-réfléchissant disposé sur une surface du système la plus proche d'un oeil d'un porteur du système.

5. Système ophtalmique (100, 200, 300, 400, 500) selon la revendication 4, où la lumière réfléchie observée par un porteur du système est inférieure à 8 %, préférentiellement inférieure à 3 %.

6. Système ophtalmique (100, 200, 300, 400, 500) selon la revendication 1, où le colorant est le pérylène, une porphyrine ou un colorant similaire, pouvant absorber des longueurs d'onde bleues.

7. Système ophtalmique (100, 200, 300, 400, 500) selon la revendication 1, où le colorant absorbe sélectivement au moins 5 % de la lumière dans une plage de longueur d'onde comprise entre 400 nm et 460 nm, et où le système est prévu pour présenter une transmission moyenne d'au moins 80 % dans le spectre visible.

8. Système ophtalmique (100, 200, 300, 400, 500) selon la revendication 7, où le système est prévu pour présenter une transmission moyenne d'au moins 90 % dans le spectre visible.

9. A procédé de réalisation d'un dispositif ophtalmique, comprenant :
la préparation d'une première couche comprenant un matériau ophtalmique et un colorant absorbant sélectivement au moins 5 % de la lumière dans une plage de longueur d'onde comprise entre 400 nm et 460 nm ;
et
le dépôt d'un film sur la première couche ;
où le film compense un déséquilibre de couleurs résultant de l'absorption de lumière par le colorant, de telle manière que la lumière blanche présente des coordonnées CIE (x, y) de (0,33±0,05, 0,33±0,05), préférentiellement de (0,33±0,02, 0,33±0,02), après transmission par le dispositif.

10. Procédé selon la revendication 9, où la plage de longueur d'onde est comprise ou est sensiblement comprise entre 420 nm et 440 nm.

11. Procédé selon la revendication 10, où la plage de longueur d'onde absorbée par le colorant inclut l'absorption d'au moins 20 %, préférentiellement d'au moins 50 % de la lumière ayant une longueur d'onde de 430 nm.

12. Procédé selon la revendication 9, où le film est un revêtement anti-réfléchissant.

13. Procédé selon la revendication 9, comprenant en outre :
le dépôt d'une deuxième couche du matériau ophtalmique sur le film, le film étant intercalé entre la première couche et la deuxième couche.

14. Système ophtalmique selon la revendication 7, où la plage de longueur d'onde couvre 60 nm, 40 nm ou 20 nm.

15. Système ophtalmique selon la revendication 7, où la largeur à mi-hauteur de la plage de longueur d'onde sélectivement absorbée est sensiblement de 15 nm.

16. Système ophtalmique selon la revendication 1 ou la revendication 7, où la plage de longueur d'onde est comprise ou est sensiblement comprise entre 420 nm et 440 nm.

17. Système ophtalmique selon la revendication 16, où la plage de longueur d'onde absorbée par le colorant inclut l'absorption d'au moins 20 %, préférentiellement d'au moins 50 % de la lumière ayant une longueur d'onde de 430 nm.
